# EUROPÄISCHE PATENTSCHRIFT

(11) **EP 0 505 519 B1**
(45) Veröffentlichungstag und Bekanntmachung des Hinweises auf die Patenterteilung: **02.08.1995**
(21) Anmeldenummer: 91915521.8
(22) Anmeldetag: 05.09.1991
(51) Int. Cl.: A61K 35/78

(54) **KAWA-KAWA-EXTRAKT, VERFAHREN ZU SEINER HERSTELLUNG UND SEINE VERWENDUNG**
KAVA EXTRACT, METHOD OF PREPARING THE EXTRACT, AND ITS USE
EXTRAIT DE KAWA-KAWA, SON PROCEDE DE PRODUCTION ET SON UTILISATION

(30) Priorität: 12.09.1990 DE 4028945
(43) Veröffentlichungstag der Anmeldung: 30.09.1992
(73) Patentinhaber: Dr. Willmar Schwabe GmbH & Co., D-76209 Karlsruhe (DE)
(72) Erfinder: SCHWABE, Klaus-Peter, D-7500 Karlsruhe 41 (DE)
(74) Vertreter: Bunke, Holger, Dr.rer.nat. Dipl.-Chem.
(86) Internationale Anmeldenummer: EP9101687
(87) Internationale Veröffentlichungsnummer: WO9204036

(56) Entgegenhaltungen:
- DE-C- 197 806
- FR-M- 7 893
- GB-A- 943 121

## Beschreibung

Die Erfindung betrifft einen flavokawinarmen Extrakt aus dem "Kawa-Kawa" genannten Rhizom von Piper methysticum Forst. (Rauschpfeffer, Familie Piperaceae) mit hohem Gehalt an Kawalactonen und guter Bioverfügbarkeit.

In Polynesien, der Heimat des Kawa-Strauches, verwenden die Eingeborenen seit jeher einen wäßrigen Auszug (Kaltmazerat) der Droge zu rituellen und therapeutischen Zwecken.

Die entspannende Wirkung der Droge, die als Phytotranquilizer zur Entspannung bei Nervosität und Überreizung und als Mittel zur Schlafinduktion Verwendung findet, beruht auf dem Vorkommen mehrerer 6-substituierter 4-Methoxypyrone, nämlich den Kawalactonen Kawain, Dihydrokawain, Methysticin, Dihydromethysticin, Yangonin und Desmethoxyyangonin (vgl. Hänsel et al., Deutsche Apothekerzeitung 125, Nr. 41, Seite 2056-2058 (1985)). Daneben sind in der Kawa-Droge die gelben Verbindungen Flavokawin A und Flavokawin B enthalten, denen hautspezifische Nebenwirkungen zugeschrieben werden (Shulgin, Bulletin on Narcotics, Vol. XXV, Nr. 2, Seite 59-74 (1973)). Schließlich enthält die Droge noch eine Reihe weiterer, noch nicht vollständig geklärter Inhaltsstoffe, die pharmakologisch inert sind und als "Matrixstoffe" bezeichnet werden.

Einige Kawalactone sind auch synthetisiert worden, darunter Kawain, dessen Razemat als Psychopharmakon in Form von Kapseln mit 200 mg des Wirkstoffs im Handel ist.

Die Kawalactone sind in Wasser praktisch unlöslich: Beispielsweise beträgt die maximale Löslichkeit von Kawain bei 21°C 2,2 mg / 100 ml Wasser. In dem von den Polynesiern verwendeten Kaltmazerat befinden sich jedoch bis zu etwa 70 mg an Kawapyronen (Gesamtpyrongehalt) in jeweils 100 ml Mazerat. Hieraus wurde geschlossen, daß die in der Droge enthaltenen, pharmakologisch inerten "Matrixsubstanzen" als Lösungsvermittler für die Kawapyrone wirken (vgl. Hänsel et al., a.a.O.).

Der mit den bisher auf dem Markt befindlichen Kawalacton-Präparaten erzielbare therapeutische Effekt ist wegen ihrer geringen Bioverfügbarkeit unbefriedigend. Die einzelnen Reinsubstanzen, gleichgültig, ob synthetisiert oder aus der Droge isoliert, müssen wegen ihrer schlechten Löslichkeit hochdosiert verabreicht werden, während die aus der Droge gewonnenen Trockenextrakte einen Gesamtlactongehalt von nur etwa 5 bis 30 Gew.% aufweisen, so daß trotz der gegenüber den Reinsubstanzen besseren Löslichkeit wiederum hochdosiert werden muß, um den gewünschten Mindesttiter im Blut bzw. Plasma zu erhalten.

Der Erfindung liegt deshalb die Aufgabe zugrunde, einen Trockenextrakt mit wesentlich höherem Gesamtlactongehalt, aber dennoch guter Wasserlöslichkeit, mit hoher Bioverfügbarkeit nach oraler Applikation und mit möglichst geringem Flavokawin-Gehalt sowie ein einfaches Verfahren zu seiner Herstellung zu schaffen. Ferner liegt der Erfindung die Aufgabe zugrunde, Arzneimittel mit hohem Gehalt an gut löslichen und bioverfügbaren Kawalactonen und möglichst niedrigem Flavokawingehalt bereitzustellen, die oral appliziert werden können und bei denen die Gefahr von Nebenwirkungen durch Verfärbung der Haut gegenüber den bisher bekannten Präparaten drastisch reduziert ist.

Diese Aufgabe wird erfindungsgemäß gelöst durch einen Trockenextrakt aus dem Rhizom von Piper methysticum Forst., der gekennzeichnet ist durch einen Gesamtgehalt an Kawalactonen von mindestens 50 Gew.% und einer Flavokawingehalt von höchstens 0,3 Gew.%. Vorzugsweise weist der Trockenextrakt einen Gesamtgehalt an Kawalactonen von 50 bis 90 Gew.% und/oder einen Flavokawingehalt von weniger als 0,3 Gew.% auf. Besonders vorteilhaft ist ein Extrakt, dessen Gesamtgehalt an Kawalactonen 60 bis 80 Gew.% und dessen Flavokawingehalt weniger als 0,2 Gew.% beträgt.

Obwohl der erfindungsgemäße Extrakt im Vergleich zu den bisher bekannten Extrakten hochangereichert ist und einen Gesamtgehalt an Kawalactonen von bis zu 90 Gew.% aufweist und somit den Reinpräparaten, die beispielsweise zu 100 % aus Kawain bestehen, sehr nahe kommt, hat sich überraschenderweise gezeigt, daß die Bioverfügbarkeit des erfindungsgemäßen Extrakts wesentlich besser ist, und zwar sowohl im Vergleich zu den bisher bekannten Trockenextrakten als auch im Vergleich zu den bisher als Arzneimittel verwendeten Reinsubstanzen, obwohl der pharmakologisch inerte Matrixanteil bis auf 20 Gew.% verringert werden konnte, während er bei den bisher bekannten Extrakten mindestens 70 Gew.% betragen hatte.

Der erfindungsgemäße Trockenextrakt kann deshalb vorteilhaft zur Herstellung von Arzneimitteln, insbesondere von Phytopharmaka, verwendet werden, da zur Erzielung des gleichen pharmakologischen Effektes eine geringere Dosierung ausreicht, als sie mit den bisher bekannten Mitteln erforderlich war. Wie klinische Prüfungen gezeigt haben, liegt die wirksame Tagesdosis bei etwa 300 mg des erfindungsgemäßen Extraktes, was etwa 50 mg Rein-Kawain entspricht, während die wirksame Tagesdosis von als Reinsubstanz verabreichtem Kawain etwa 200 mg beträgt (vgl. Kretschmer, "Kavain als Psychopharmakon", MMW 4/1970, Seite 154-158).

Der erfindungsgemäße Trockenextrakt kann auf einfache Weise dadurch hergestellt werden, daß das pulverisierte Rhizom von Piper methysticum Forst. (Rhizoma Kawa-Kawa) mit einem geeigneten Lösungsmittel wie Aceton, Chloroform, Ethylacetat, Niedrigalkanole mit 1 bis 4 C-Atomen oder deren mindestens 50 gew.%ige Gemische mit Wasser extrahiert und die Extraktlösung zur Trockne eingeengt wird. Die Extraktion kann in Form einer Perkolation oder einer mehrstufigen Rühr- oder Wirbelextraktion bei Raumtemperatur oder bei erhöhter Temperatur durchgeführt werden. Je nach dem verwendeten Lösungsmittel weisen die so erhaltenen Rohextrakte einen Gehalt an Kawalactonen von etwa 50 bis 70 Gew.% auf.

Anschließend werden die Rohextrakte erneut in Lösung gebracht und durch Kältefällung aus mit Wasser mischbaren Lösungsmitteln, insbesondere Ethanol und Aceton, oder durch Lösungsmittelverteilung zwischen einer wäßrigen, wäßrig-alkoholischen oder wäßrig-acetonischen ersten Phase und einer mit dieser ersten Phase nicht mischbaren zweiten Phase gereinigt, wobei die zweite Phase eine aus organischen Lösungsmitteln wie Chloroform, Heptan, Hexan, Essigsäureethylester und dergleichen sowie deren Gemischen gebildete organische Phase ist. Der Gesamtgehalt der so erhaltenen Reinextrakte an Kawalactonen gegenüber den Ausgangsstoffen ist nur wenig verringert, während der Flavokawingehalt drastisch auf ein Drittel bis ein Fünftel des Ausgangsgehalts reduziert ist.

Bei der Kältefällung wird als Lösungsmittel zunächst ein mit Wasser mischbares organisches Lösungsmittel wie Ethanol oder ein anderes Niedrigalkanol oder Aceton verwendet, und die aufkonzentrierte Lösung wird mit Wasser oder mit einem Alkanol-Wasser-Gemisch bis zum Einsetzen einer milchigen Trübung versetzt und durchmischt, wonach das Gemisch - vorzugsweise auf 5 bis 10° - abgekühlt und gegebenenfalls mehrere Stunden, vorzugsweise etwa 12 bis 24 Stunden, stehen gelassen wird. Danach wird ausgefallenes Präzipitat abgetrennt und der Überstand zur Trockne eingeengt.

Bei Anwendung der Lösungsmittelverteilung werden die Ausgangsstoffe entweder in einem organischen, mit Wasser nicht mischbaren Lösungsmittel wie z.B. Chloroform, Hexan, Heptan oder einem Essigsäureethylester/Hexan-Gemisch gelöst, wonach diese organische Phase portionsweise mit kleinen Mengen einer wäßrigen, wäßrig-alkoholischen oder wäßrig-acetonischen Phase, z.B. Wasser, Ethanol/Wasser oder Aceton/Wasser, ausgeschüttelt wird. Oder die Ausgangsstoffe werden in einem mit Wasser mischbaren organischen Lösungsmittel, beispielsweise Ethanol oder Aceton, gelöst und die nach Zugabe von Wasser wäßrig-alkoholische oder wäßrig-acetonische Phase wird mit kleinen Mengen eines organischen, mit Wasser nicht mischbaren Lösungsmittels mehrfach ausgeschüttelt bzw. ausgerührt.

In jedem Falle befinden sich die Kawalactone in der wäßrigen, wäßrig-alkoholischen bzw. wäßrig-acetonischen Phase, während die Flavokawine sich in der organischen, mit Wasser nicht mischbaren Phase anreichern und durch Abtrennen dieser organischen Phase entfernt werden können.

Die Reinextrakte sind von sirupöser, öliger oder harziger Konsistenz. Zur Herstellung von Tabletten, Dragees oder Kapseln werden sie mit üblichen Hilfsstoffen, z.B. feinteiliger Kieselsäure wie Aerosil , vermengt und vakuumgetrocknet.

Die Erfindung wird anhand der folgenden Ausführungsbeispiele weiter erläutert. In der Zeichnung sind:
- Fig. 1 und 2: Stoffverteilungsdiagramme, die im Zusammenhang mit Beispiel 2 näher erläutert sind,

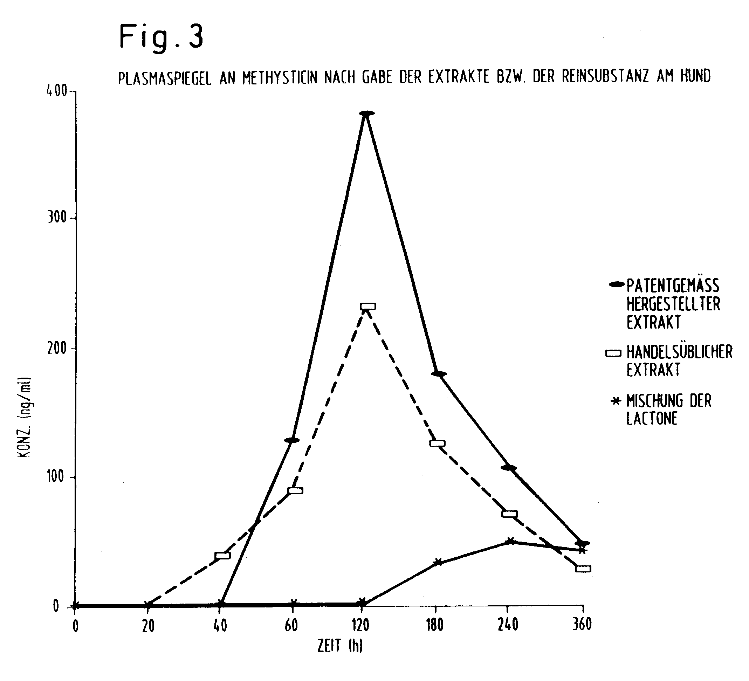

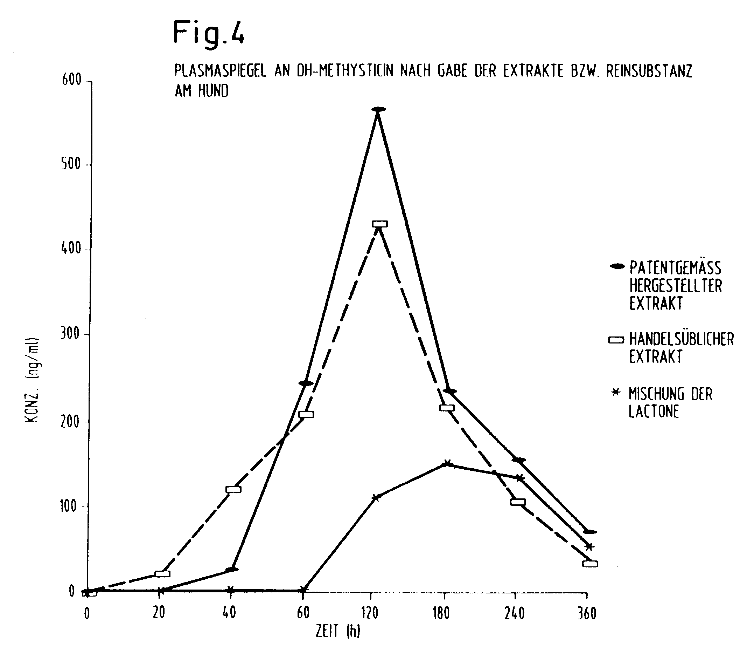

- Fig. 3 und 4: Diagramme, die den zeitlichen Verlauf des Plasmaspiegels von Methysticin und Dihydromethysticin beim Hund wiedergeben, und
- Fig. 5: eine räumliche Darstellung des Hamilton-Anxiety-Score mit Placebo-Vergleich.

### Beispiel 1

a) Extraktion mit Aceton:
1 kg gemahlene Kawa-Kawa-Droge wird mit der 5-fachen Gewichtsmenge Aceton versetzt, homogenisiert und in einen Extraktionskolben übergeführt. Bei 60°C Wasserbad-Temperatur wird 1 Stunde unter Rückfluß extrahiert. Nach dem Abkühlen wird die Extraktlösung über ein Faltenfilter filtriert. Das Filtrat wird am Rotationsverdampfer bei 60°C Wasserbad-Temperatur zur Trockne (ölig) eingeengt. Der Filterrückstand kann gegebenenfalls ausgepreßt und erneut extrahiert werden.
Die Ausbeute beträgt 27,68 g öligen Extrakts mit einem Gehalt an Kawa-Lactonen von 66 %.
b) Abreicherung von gelben Farbstoffen (Flavokawinen) durch Kombination von Lösungsmittel/Kältefällung
5 g des durch die vorstehend beschriebene Extraktion gewonnenen Kawaextrakts werden in 100 ml Ethanol (96 %) vollständig gelöst (A). Außerdem werden 10 g des Kawaextrakts in 100 ml Aceton vollständig gelöst (B). Jeweils 20 ml dieser Ausgangslösungen werden mit Wasser ad 50 ml verdünnt, wobei eine milchige Färbung auftritt.
Die wäßrigen Verdünnungen werden 12 Stunden auf 5 bis 10°C gekühlt.
Anschließend erfolgt Klärung durch Zentrifugation oder Filtration und die Bestimmung der Kawa-Lactone und Flavokawine im Überstand der Fällung.

**Tabelle 1**

| | Ausgangsgehalt | Gehalt nach Lösungsmittel/Kältefällung |
|---|---|---|
| Kawalactone | 100 % | 93 % (A) |
| Flawokawine | 100 % | 27 % (A) |
| Kawalactone | 100 % | 87 % (B) |
| Flavokawine | 100 % | 20 % (B) |

Unter diesen Bedingungen resultiert eine Ausfällung und Abreicherung der Flavokawine auf ca. 20 % des Ausgangswertes. Der Verlust an Kawa-Lactonen liegt bei ca. 10 %.

### Beispiel 2

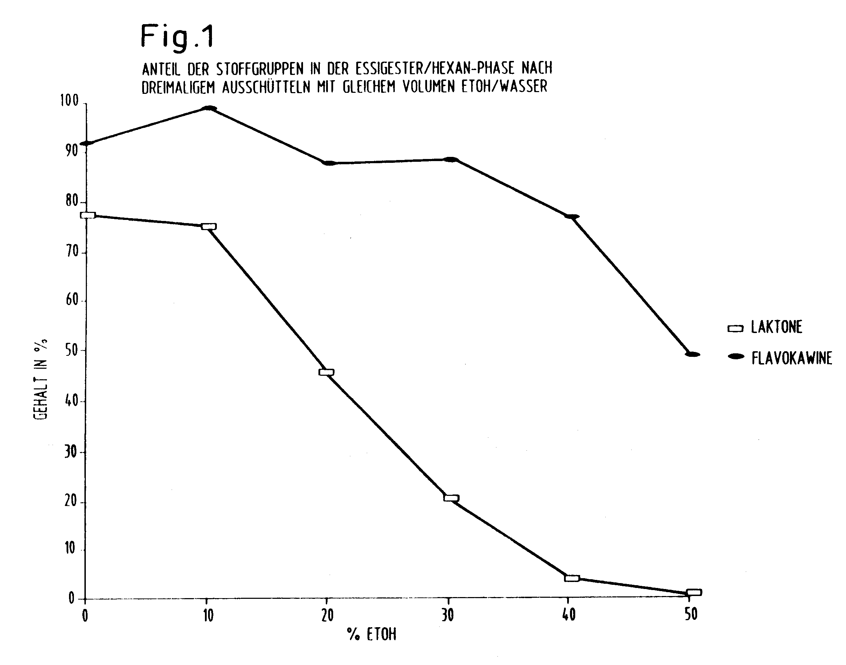

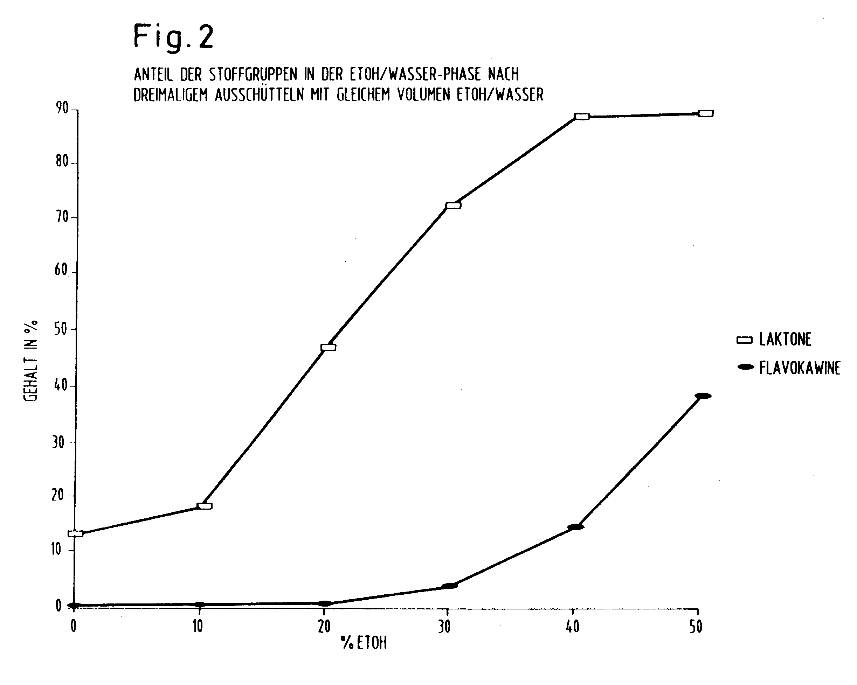

a) Extraktion mit 95 % Äthanol:
   500 g gemahlene Kawa-Kawa-Droge wird mit 4000 g 95 %-Äthanol versetzt, homogenisiert, in einen Extraktionskolben übergeführt und 1 Stunde bei 60°C Wasserbad-Temperatur extrahiert. Nach dem Abkühlen wird über eine Filternutsche filtriert. Der Drogenrückstand wird noch einmal auf die gleiche Weise extrahiert und filtriert. Die Extraktlösungen werden vereinigt und an Rotationsverdampfer bei 60°C Wasserbad-Temperatur zur Trockne eingeengt.
   - Ausbeute:: 28,88 g Kawa-Extrakt mit einem Gehalt an Kawa-Lactonen von 43 %.
b) Abreicherung von gelben Farbstoffen (Flavokawinen) durch Lösungsmittelverteilung
   Es werden gesättigte Lösungen des so gewonnenen Kawa-Extrakts in 30 ml eines Gemisches von 5 Volumenteilen Essigsäureethylester und 25 Volumenteilen n-Hexan hergestellt.
   Diese Ansätze werden je 3-mal mit je 30 ml Ethanol/Wasser (Ethanolanteil zwischen 20 bis 50 % (v/v)) ausgeschüttelt.
   In der Ethanol/Wasserphase bzw. der Hexan/Essigesterphase werden die Stoffmengen an Kawa-Lactonen und Flavokawinen durch HPLC-Analyse bestimmt.
   Aus den Diagrammen der Stoffverteilung (Fig. 1 und 2) ist zu entnehmen, daß insbesondere bei einem Ethanol-Anteil von 30 bis 40 % die Flavokawine auf unter 20 % des Ausgangswertes abgereichert werden. Die Kawa-Lactone werden unter diesen Bedingungen zu mehr als 70 bis 80 % zurückgewonnen.

### Beispiel 3

a) Extraktion mit Chloroform:
500 g Kawa-Kawa-Droge werden mit der 10-fachen Gewichtsmenge Chloroform in einem 6 l-Dreihalskolben unter Rühren 2 Stunden am Rückfluß extrahiert.
Nach dem Abkühlen wird über eine große Nutsche abgesaugt. Der Drogenrückstand wird noch 2-mal auf die gleiche Weise extrahiert. Die Chloroform-Extraktlosungen werden vereinigt und am Rotationsverdampfer bei ca. 50°C Bad-Temperatur bis zur öligen Konsistenz eingeengt.
- Ausbeute:: 12,90 g Kawa-Extrakt mit einem Gehalt an Kawa-Lactonen von 63 %.

b) Abreicherung von gelben Farbstoffen (Flavokawinen) durch Lösungsmittelverteilung
Aus dem so gewonnenen Kawa-Extrakt werden gesättigte Lösungen in je 50 ml von Ethanol/Wasser-Mischungen hergestellt (jeweils ca. 1-2 g).
Diese Ansätze werden je 3-mal mit gleichem Volumen Hexan oder Heptan extrahiert. In der Ethanol/Wasser-Phase bzw. der Hexanphase werden die Anteile an Kawalactonen und Flavokawinen ermittelt. Die Produktphase (EtOH/Wasser) wies die in Tabelle 2 angegebenen Gehalte an Kawalactonen und Flavokawinen (jeweils bezogen auf Ausgangsextrakt) auf:

**Tabelle 2**

| | Kawalactone | Flavokawine |
|---|---|---|
| Ethanol/H₂O 85:15 (v/v) | 83 % | 54 % |
| Ethanol/H₂O 70:30 | 85 % | 40 % |
| Ethanol/H₂O 50:50 | 73% | 8 % |

Es ist ersichtlich, daß bei einem Ethanolgehalt zwischen 50 und 70 % eine starke Abreicherung der Flavokawine bei vertretbarem Verlust an Kawalactonen auftritt.

### Beispiel 4

### Abreicherung von Flavokawinen durch Verteilung zwischen Aceton/Wasser und Heptan

Ein entsprechend Beispiel 1 hergestellter acetonischer Extrakt wird zur Trockne eingeengt und danach wieder gelöst in 55 Gew.% Aceton/Wasser zu einer Lösung mit 4 % Trocknungsrückstand. Diese Lösung wird in einem 4-stufigen Mixer-Settler im Gegenstrom mit der gleichen Volumenmenge eines Gemisches aus 8 Teilen Heptan und 2 Teilen Aceton extrahiert.

Die im Trockenrückstand der Aceton/Wasser-Phase gefundenen Analysenwerte sind nachstehend den Ausgangswerten gegenübergestellt:

| | Kawalactone | Flavokawine |
|---|---|---|
| Ausgangswerte (Extrakt ohne Heptan/Aceton-Extraktion) | 67,63 % | 1,89 % |
| Aceton-Wasser-Phase | 70,66 % | 0,17 % |

Die Erfindung wird hinsichtlich der pharmakokinetischen Eigenschaften der Kawa-Extrakte und der daraus hergestellten Arzneimittel weiter wie folgt erläutert:

Die Bioverfügbarkeiten von Kawa-Lactonen beim Hund werden nach Gabe des erfindungsgemäß hergestellten Extraktes, eines handelsüblichen Extraktes sowie eines Gemisches der Kawa-Lactone als Reinsubstanzen miteinander verglichen.

Der erfindungsgemäß hergestellte Extrakt wurde mit einem galenischen Hilfsstoff (Siliciumdioxid) vermischt und in Form einer Kapsel verabreicht, ebenso wurde der mit einem galenischen Hilfsstoff (Siliciumdioxid) vermischte handelsübliche Extrakt verabreicht. Die Mischung der Kawa-Lactone erfolgte in gleichen Teilen, wie beim erfindungsgemäß hergestellten Extrakt, die Verabreichung erfolgte ebenfalls als Kapsel. Die Dosierung ist Tabelle 3 zu entnehmen.

**Tabelle 3**

| Lacton | Dosierung (mg/kg) | | |
|---|---|---|---|
| | erfindungsgemäß hergestellter Extrakt | Mischung der Reinsubstanzen | handelsüblich hergestellter Extrakt |
| Kawain | 1,23 | 1,23 | 1,23 |
| DH-Kawain | 1,43 | 1,43 | 1,34 |
| Methysticin | 1,14 | 1,14 | 1,25 |
| DH-Methysticin | 1,23 | 1,23 | 1,30 |
| Yangonin | 0,82 | 0,82 | 0,94 |

Blut wurde zu folgenden Zeitpunkten nach Verabreichung abgenommen: 10, 20, 40, 60, 120, 180, 240 und 360 Minuten. Die Kawa-Lactone wurden im Plasma durch HPLC bestimmt.

Es ergaben sich folgende AUC-Werte¹⁾ (0-6 h) an je 5 Hunden (Mittelwerte ± Standardabweichung) (Tabelle 4):
¹⁾ AUC = area under the curve, berechnet nach der Trapezregel; vgl. Derendorf, Garret, "Pharmakokinetik", Wissenschaftliche Verlagsgesellschaft mbH, Stuttgart 1987

**Tabelle 4**

| Lacton | AUC (ng/ml·min) | |
|---|---|---|
| | erfindungsgemäß hergestellter Extrakt | Mischung der Reinsubstanzen |
| Kawain | 22210 ± 5862 | - |
| DH-Kawain²⁾ | 4144 ± 4134 | - |
| Methysticin | 26598 ± 13956* | 4907 ± 3329 |
| DH-Methysticin²⁾ | 58534 ± 12626** | 20686 ± 9334 |
| Yangonin | - | - |
| - Plasmaspiegel unter der Nachweisgrenze (ca. 30 ng/ml Lacton) | | |

| | | |
|---|---|---|
| Signifikanzniveau * p < 0,05 | | |
| ** p < 0,01 | | |
| ²⁾ DH = Dihydro | | |

Es zeigte sich, daß nur der erfindungsgemäß hergestellte Extrakt zu einer meßbaren Resorption von Kawain und DH-Kawain führt. Die Resorption von Methysticin und DH-Methysticin sind beim erfindungsgemäß hergestellten Extrakt signifikant besser.

In Tabelle 5 sind die AUC-Werte von vier Hunden aufgeführt, die im cross-over jeweils den erfindungsgemäß hergestellten Extrakt sowie einen handelsüblichen Extrakt erhielten.

**Tabelle 5**

| Substanz | Tier | AUC (ng/ml.min) | | Quotient AUC_{A}/AUC_{B} |
|---|---|---|---|---|
| | | erfindungsgemäß hergest. Extrakt A | handelsüblich hergestellter Extrakt* B | |
| Kawain | 1 | 32.080 | 30.855 | 1,04 |
| | 2 | 19.540 | 24.430 | 0,80 |
| | 3 | 22.710 | 17.325 | 1,31 |
| | 4 | 19.590 | 18.303 | 1,07 |
| DH-Kawain | 1 | 4.200 | - | - |
| | 2 | - | 3.970 | - |
| | 3 | 2.700 | 4.226 | 0,63 |
| | 4 | 11.020 | 1.153 | 9,56 |
| Methysticin | 1 | 51.070 | 30.826 | 1,66 |
| | 2 | 22.320 | 12.476 | 1,79 |
| | 3 | 21.810 | 7.620 | 2,86 |
| | 4 | 22.080 | 9.275 | 2,38 |
| DH-Methysticin | 1 | 76.480 | 58.496 | 1,31 |
| | 2 | 52.830 | 41.962 | 1,26 |
| | 3 | 63.720 | 21.762 | 2.93 |
| | 4 | 57.040 | 33.267 | 1,71 |

| | | | | |
|---|---|---|---|---|
| * auf gleiche Dosis normiert | | | | |

Ein Vergleich der relativen Bioverfügbarkeit des erfindungsgemäß hergestellten Extraktes mit dem handelsüblichen Extrakt wurde an Hand der Bioverfügbarkeitsquotienten (Quotient AUC_{A}/AUC_{B}) durchgeführt. Von Bioäquivalenz wird gesprochen, wenn das 95 %-Konfidenzintervall der Bioverfügbarkeitsquotienten zwischen 0,8 und 1,20 liegt bzw. wenn 3/4 der Werte zwischen 0,75 und 1,25 liegen (vgl. H. Blume, "Bioäquivalenz-Beurteilungsgrundlage für Generika", Pharmazeutische Zeitung 132, Nr. 4, Seiten 151-162 (1987)).

Für Methysticin und DH-Methysticin liegen sämtliche Bioverfügbarkeitsquotienten außerhalb des Bereiches von 0,75 und 1,25 und betragen im Mittel 2,17 bzw. 1,80. Das heißt, die bessere Bioverfügbarkeit des erfindungsgemäß hergestellten Extraktes gegenüber dem handelsüblich hergestellten Extrakt ist nach anerkannten Kriterien gesichert. Beispielhaft sind die Plasmaspiegel von Methysticin und DH-Methysticin nach Gabe der verschiedenen Extrakte bzw. Reinsubstanzen an einem Hund zu sehen.

Bei Kawain ist von gleicher Resorption nach Gabe beider Extrakte auszugehen.

In einer randomisierten, placebokontrollierten Doppelblindstudie wurde die Wirksamkeit des erfindungsgemäß hergestellter Extraktes bei 40 Patientinnen mit psychovegetativen und psychosomatischen Störungen untersucht.

Die Prüfung dauerte insgesamt acht Wochen. Die Patientinnen erhielten während dieser Zeit entweder 3 x 1 Kapsel à 100 mg Extrakt oder Placebo.

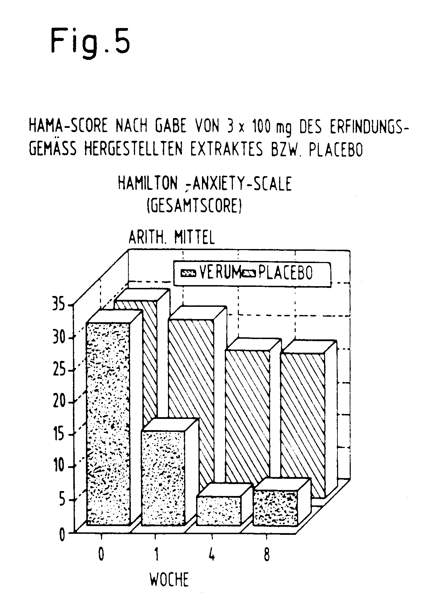

Die Wirksamkeit wurde an Hand des Hamilton-Anxiety-Scores beurteilt (HAMA). Nach Gabe des erfindungsgemäß hergestellten Extraktes sinkt in der vierten und achten Behandlungswoche der HAMA-Score signifikant ab (p < 0,01 bzw. 0,05), wie aus der Graphik (Fig. 5) ersichtlich.

## Patentansprüche (Patentansprüche für folgende(n) Vertragsstaat(en): AT, BE, CH, LI, DE, DK, FR, GB, GR, IT, LU, NL, SE)

1. Trockenextrakt aus dem Rhizom von Piper methysticum Forst., **gekennzeichnet durch** einen Gesamtgehalt an Kawa-Lactonen von mindestens 50 Gew.% und einen Flavokawingehalt von höchstens 0,3 Gew.%.

2. Extrakt nach Anspruch 1, **gekennzeichnet durch** einen Gesamtgehalt an Kawa-Lactonen von 50 bis 90 Gew.%.

3. Extrakt nach Anspruch 1 oder 2, **gekennzeichnet durch** einen Flavokawingehalt von weniger als 0,3 Gew.%.

4. Extrakt nach einem der Ansprüche 1 bis 3, **gekennzeichnet durch** einen Gesamtgehalt an Kawa-Lactonen von 60 bis 80 Gew.% und einen Flavokawingehalt von weniger als 0,2 Gew.%.

5. Verfahren zur Herstellung des Trockenextrakts gemäß einem der Ansprüche 1 bis 4, **dadurch gekennzeichnet**, daß das pulverisierte Rhizom von Piper methysticum Forst. (Rhizoma Kawa-Kawa) mit einem geeigneten Lösungsmittel - wie Aceton, Chloroform, Ethylacetat, Niedrigalkanole mit 1 bis 4 C-Atomen oder deren mindestens 50 gew.%ige Gemische mit Wasser - extrahiert und die Extraktlösung zur Trockne (Rohextrakt) eingeengt wird und daß der Rohextrakt erneut in Lösung gebracht und der Gehalt an Flavokawinen durch Kältefällung oder durch Lösungsmittelverteilung abgereichert wird, wonach die Lösung zur Trockne (Reinextrakt) eingeengt wird.

6. Verfahren nach Anspruch 5, **dadurch gekennzeichnet**, daß die Kältefällung durchgeführt wird, indem die Rohextrakt-Lösung mit Wasser oder mit einem Alkanol/Wasser-Gemisch versetzt und durchmischt wird, wonach das Gemisch auf 5 bis 10°C abgekühlt und mehrere Stunden stehengelassen wird.

7. Verfahren nach Anspruch 5, **dadurch gekennzeichnet**, daß die Lösungsmittelverteilung so durchgeführt wird, daß die Rohextrakt-Lösung durch Zugabe geeigneter Lösungsmittel auf zwei nicht mischbare, getrennte Phasen, eine organische und eine wäßrige Phase, verteilt wird, die wäßrige Phase abgetrennt und zur Trockne eingeengt wird.

8. Verwendung des Trockenextrakts gemaß einem der Ansprüche 1 bis 4 zur Herstellung von Arzneimitteln, insbesondere von Phytopharmaka.

9. Arzneimittel, enthaltend einen Extrakt gemäß einem der Ansprüche 1 bis 4 sowie gegebenenfalls übliche Hilfs- und Zusatzstoffe.

## Patentansprüche (Patentansprüche für folgende(n) Vertragsstaat(en): ES)

1. Verfahren zur Herstellung eines Trockenextrakts aus dem Rhizom von Piper methysticum Forst. mit einem Gesamtgehalt an Kawa-Lactonen von mindestens 50 Gew.% und einem Flavokawingehalt von höchstens 0,3 Gew.%, dadurch gekennzeichnet, daß das pulverisierte Rhizom von Piper methysticum Forst. (Rhizoma Kawa-Kawa) mit einem geeigneten Lösungsmittel - wie Aceton, Chloroform, Ethylacetat, Niedrigalkanole mit 1 bis 4 C-Atomen oder deren mindestens 50 gew.%ige Gemische mit Wasser - extrahiert und die Extraktlösung zur Trockne (Rohextrakt) eingeengt wird und daß der Rohextrakt erneut in Lösung gebracht und der Gehalt an Flavokawinen durch Kältefällung oder durch Lösungsmittelverteilung abgereichert wird, wonach die Lösung zur Trockne (Reinextrakt) eingeengt wird.

2. Verfahren nach Anspruch 1, dadurch gekennzeichnet, daß die Kältefällung durchgeführt wird, indem die Rohextrakt-Lösung mit Wasser oder mit einem Alkanol/Wasser-Gemisch versetzt und durchmischt wird, wonach das Gemisch auf 5 bis 10°C abgekühlt und mehrere Stunden stehengelassen wird.

3. Verfahren nach Anspruch 1, dadurch gekennzeichnet, daß die Lösungsmittelverteilung so durchgeführt wird, daß die Rohextrakt-Lösung durch Zugabe geeigneter Lösungsmittel auf zwei nicht mischbare, getrennte Phasen, eine organische und eine wäßrige Phase, verteilt wird, die wäßrige Phase abgetrennt und zur Trockne eingeengt wird.

4. Verfahren nach einem der Ansprüche 1 bis 3 zur Herstellung eines Trockenextrakts mit einem Gesamtgehalt an Kawa-Lactonen von 50 bis 90 Gew.%.

5. Verfahren nach einem der Ansprüche 1 bis 4 zur Herstellung eines Trockenextrakts mit einem Flavokawingehalt von weniger als 0,3 Gew.%.

6. Verfahren nach einem der Ansprüche 1 bis 5 zur Herstellung eines Trockenextrakts mit einem Gesamtgehalt an Kawa-Lactonen von 60 bis 80 Gew.% und einem Flavokawingehalt von weniger als 0,2 Gew.%.

## Claims (Claims for the following Contracting State(s): AT, BE, CH, LI, DE, DK, FR, GB, GR, IT, LU, NL, SE)

1. Dry extract from the rhizome of Piper methysticum Forst., characterized by a total content of kava lactones of at least 50 % by weight and a flavokawin content of at the most 0.3 % by weight.

2. Extract according to claim 1, characterized by a total content of kava lactones of 50 to 90 % by weight.

3. Extract according to claim 1 or 2, characterized by a flavokawin content of less than 0.3 % by weight.

4. Extract according to any one of claims 1 to 3, characterized by a total content of kava lactones of 60 to 80 % by weight and a flavokawin content of less than 0.2 % by weight.

5. Process for the preparation of the dry extract according to any one of claims 1 to 4, characterized in that the pulverized rhizome of Piper methysticum Forst. (rhizoma kava-kava) is extracted with a suitable solvent, such as acetone, chloroform, ethyl acetate, low alkanols having 1 to 4 C atoms or at least 50 % by weight mixtures thereof with water and the extract solution concentrated to dryness (raw extract) and that the raw extract is again brought into solution and the content of flavokawins is depleted by cold precipitation or by solvent distribution, whereafter the solution is concentrated to dryness (pure extract).

6. Process according to claim 5, characterized in that the cold precipitation is carried out in that water or an alkanol/water mixture is added to the raw extract solution and mixed therewith, whereafter the mixture is cooled to 5 to 10°C and allowed to stand for several hours.

7. Process according to claim 5, characterized in that the solvent distribution is carried out in such a manner that the raw extract solution is distributed by addition of suitable solvents amongst two immiscible separate phases, an organic and an aqueous phase, and the aqueous phase is separated and concentrated to dryness.

8. Use of the dry extract according to any one of claims 1 to 4 for the preparation of pharmaceutical preparations, in particular phytopharmaceuticals.

9. Pharmaceutical preparation containing an extract according to any one of claims 1 to 4 and possibly conventional adjuvants and additives.

## Claims (Claims for the following Contracting State(s): ES)

1. Process for the preparation of a dry extract from the rhizome of Piper methysticum Forst. having a total content of kava lactones of at least 50 % by weight and a flavokawin content of at the most 0.3 % by weight, characterized in that the pulverized rhizome of Piper methysticum Forst. (rhizoma kava-kava) is extracted with a suitable solvent, such as acetone, chloroform, ethyl acetate, low alkanols having 1 to 4 C atoms or at least 50 % by weight mixtures thereof with water and the extract solution concentrated to dryness (raw extract) and that the raw extract is again brought into solution and the content of flavokawins is depleted by cold precipitation or by solvent distribution, whereafter the solution is concentrated to dryness (pure extract).

2. Process according to claim 1, characterized in that the cold precipitation is carried out in that water or an alkanol/water mixture is added to the raw extract solution and mixed therewith, whereafter the mixture is cooled to 5 to 10°C and allowed to stand for several hours.

3. Process according to claim 1, characterized in that the solvent distribution is carried out in such a manner that the raw extract solution is distributed by addition of suitable solvents amongst two immiscible separate phases, an organic and an aqueous phase, and the aqueous phase is separated and concentrated to dryness.

4. Process according to any one of claims 1 to 3 for the preparation of a dry extract having a total content of kava lactones of 50 to 90 % by weight.

5. Process according to any one of claims 1 to 4 for the preparation of a dry extract having a flavokawin content of less than 0.3 % by weight.

6. Process according to any one of claims 1 to 5 for the preparation of a dry extract having a total content of kava lactones of 60 to 80 % by weight and a flavokawin content of less than 0.2 % by weight.

## Revendications (Revendications pour l'(les) Etat(s) contractant(s) suivant(s): AT, BE, CH, LI, DE, DK, FR, GB, GR, IT, LU, NL, SE)

1. Extrait sec du rhizome de Piper methysticum Forst., caractérisé en ce que la teneur totale en kawalactones est d'au moins 50 % en poids et en ce que la teneur en flavokawine est de 0,3 % en poids maximum.

2. Extrait selon la revendication 1, caractérisé en ce que la teneur totale en kawalactones est de 50 à 90 % en poids.

3. Extrait selon la revendication 1 ou la revendication 2, caractérisé en ce que la teneur en flavokawine est inférieure à 0,3 % en poids.

4. Extrait selon l'une quelconque des revendications 1 à 3, caractérisé en ce que la teneur totale en kawalactones est de 60 à 80 % en poids et que la teneur en flavokawine est inférieure à 0,2 % en poids.

5. Procédé de fabrication d'un extrait sec selon l'une quelconque des revendications 1 à 4, caractérisé en ce que le rhizome pulvérisé de Piper methysticum Forst. (Rhizome Kawa-Kawa) est extrait à l'aide d'un solvant approprié tel que l'acétone, le chloroforme, l'acétate d'éthyle, les alkanols inférieurs contenant de 1 à 4 atomes de carbone ou leurs mélanges à 50 % en poids au moins avec de l'eau, la solution d'extrait étant concentrée jusqu'à siccité (extrait brut), et que l'extrait brut est à nouveau mis en solution, la teneur en flavokawines étant abaissée par précipitation à froid ou par partition de solvant, la solution étant ensuite concentrée jusqu'à siccité (extrait pur).

6. Procédé, selon la revendication 5, caractérisé en ce que la précipitation à froid s'effectue en traitant et en mélangeant la solution d'extrait brut avec de l'eau ou avec un mélange d'alkanol et d'eau, le mélange obtenu étant ensuite refroidi à 5 à 10°C et laissé au repos pendant plusieurs heures.

7. Procédé, selon la revendication 5, caractérisé en ce que la partition de solvant s'effectue de manière à ce que, par addition d'un solvant approprié, la solution d'extrait brut soit divisée en deux phases séparées, non miscibles, à savoir une phase organique et une phase aqueuse, la phase aqueuse étant séparée et concentrée à siccité.

8. Utilisation d'extrait sec, selon l'une quelconque des revendications 1 à 4, pour la fabrication de médicaments, plus particulièrement de produits phytopharmaceutiques.

9. Médicaments contenant un extrait selon l'une quelconque des revendications 1 à 4, ainsi que, s'il y a lieu, des adjuvants et des additifs habituellement utilisés.

## Revendications (Revendications pour l'(les) Etat(s) contractant(s) suivant(s): ES)

1. Procédé de fabrication d'un extrait sec du rhizome de Piper methysticum Forst., comprenant une teneur totale en kawalactones d'au moins 50 % en poids et une teneur en flavokawine de 0,3 % en poids maximum, caractérisé en ce que le rhizome pulvérisé de Piper methysticum Forst. (Rhizome Kawa-Kawa) est extrait à l'aide d'un solvant approprié tel que l'acétone, le chloroforme, l'acétate d'éthyle, les alkanols inférieurs contenant de 1 à 4 atomes de carbone ou leurs mélanges à 50 % en poids au moins avec de l'eau, la solution d'extrait étant concentrée jusqu'à siccité (extrait brut), et que l'extrait brut est à nouveau mis en solution, la teneur en flavokawines étant abaissée par précipitation à froid ou par partition de solvant, la solution étant ensuite concentrée jusqu'à siccité (extrait pur).

2. Procédé, selon la revendication 1, caractérisé en ce que la précipitation à froid s'effectue en traitant et en mélangeant la solution d'extrait brut avec de l'eau ou avec un mélange d'alkanol et d'eau, le mélange obtenu étant ensuite refroidi à 5 à 10°C et laissé au repos pendant plusieurs heures.

3. Procédé, selon la revendication 1, caractérisé en ce que la partition de solvant s'effectue de manière à ce que, par addition d'un solvant approprié, la solution d'extrait brut soit divisée en deux phases séparées, non miscibles, à savoir une phase organique et une phase aqueuse, la phase aqueuse étant séparée et concentrée à siccité.

4. Procédé, selon une des revendications 1 à 3, de fabrication d'un extrait sec comprenant une teneur totale en kawalactones de 50 à 90 % en poids.

5. Procédé, selon une des revendications 1 à 4, de fabrication d'un extrait sec comprenant une teneur en flavokawine inférieure à 0,3 % en poids.

6. Procédé, selon une des revendications 1 à 5, de fabrication d'un extrait sec comprenant une teneur totale en kawalactones de 60 à 80 % en poids et une teneur en flavokawine inférieure à 0,2 % en poids.
